(19) Europäisches Patentamt
European Patent Office
Office européen des brevets

(11) Veröffentlichungsnummer: **0 049 407**
**B1**

(12) # EUROPÄISCHE PATENTSCHRIFT

(45) Veröffentlichungstag der Patentschrift: **08.05.85**

(21) Anmeldenummer: **81107423.6**

(22) Anmeldetag: **18.09.81**

(51) Int. Cl.⁴: **C 07 D 471/04,** A 61 K 31/44, C 12 P 17/18 // (C07D471/04, 235:00, 221:00)

(54) **6-Hydroxy-2-phenyl-imidazo(4,5-b)pyridine, ihre Herstellung und diese enthaltende Arzneimittel.**

(30) Priorität: **03.10.80 DE 3037464**

(43) Veröffentlichungstag der Anmeldung: **14.04.82 Patentblatt 82/15**

(45) Bekanntmachung des Hinweises auf die Patenterteilung: **08.05.85 Patentblatt 85/19**

(84) Benannte Vertragsstaaten: **BE CH DE FR IT LI LU NL SE**

(56) Entgegenhaltungen:
**EP-A-0 022 495**
**DE-A-2 305 339**
**DE-A-2 361 757**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Diederen, Willi, Dr., Haldenstrasse 1a, D-7950 Biberach 1 (DE)**
Erfinder: **Prox, Axel, Prof. Dr. Dipl.-Chem., Kapellenweg 13, D-7950 Biberach 1 (DE)**
Erfinder: **Reuter, Albert, Dr. Dipl.-Chem., Eggelspachsteig 1, D-7950 Biberach 1 (DE)**
Erfinder: **Roth, Willy, Dr. Dipl.-Chem., Amriswilstrasse 7, D-7950 Biberach 1 (DE)**
Erfinder: **Schmid, Jochen, Dr. Dipl.-Chem., Panoramaweg 19, D-7951 Warthausen (DE)**

**EP 0 049 407 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der DE-OS 2 305 339 wird u. a. die Verbindung 2-(2-Methoxy-4-methylsulfinyl-phenyl)-1H-imidazo[4,5-b]pyridin beschrieben, welche neben einer milden blutdrucksenkenden und milden herzfrequenzsteigernden Wirkung insbesondere eine positiv inotrope Wirkung aufweist.

Es wurde nun gefunden, daß die neuen 6-Hydroxy-2-phenyl-imidazo-[4,5-b]pyridine der allgemeinen Formel

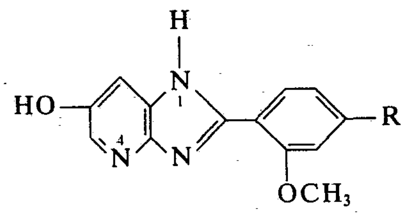

(I)

in der

R eine Methylsulfinyl- oder Methylsulfonylgruppe darstellt,

deren 3H-Tautomere und deren physiologisch verträgliche Säureadditionssalze mit anorganischen und organischen Säuren bei einer geringeren herzfrequenzsteigernden und keiner blutdrucksenkenden Wirkung ebenfalls positiv inotrope Wirkungen aufweisen.

Die neuen Verbindungen sind daher insbesondere zur Behandlung bei Patienten mit Low-output Syndrom und bei Patienten mit ischämisch bedingter Herzinsuffizienz geeignet.

Gegenstand der Erfindung sind daher die neuen 6-Hydroxy-2-phenyl-imidazo[4,5-b]pyridine der obigen allgemeinen Formel I, diese sie enthaltende Arzneimittel und Verfahren zu ihrer Herstellung.

Erfindungsgemäß erhält man die Verbindungen der allgemeinen Formel I durch Kernhydroxylierung einer Verbindung der allgemeinen Formel

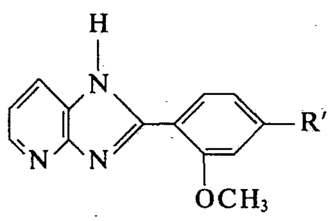

(II)

in der

R′ eine Methylmercapto-, Methylsulfinyl- oder Methylsulfonylgruppe darstellt.

Die Kernhydroxylierung wird vorzugsweise in einem Lösungsmittel wie Wasser, tert. Butanol oder einem Polyäthylenglykoläther wie Dimethoxydiäthylenglycol oder deren Gemischen in Gegenwart eines Oxidationsmittels, z. B. einer Peroxyverbindung wie Wasserstoffperoxid, Perschwefelsäure, Perdischwefelsäure oder eines Oxidationsmittels wie Chromsäure, einer $Ce^{IV}$-, $V^{V}$- oder $Mn^{VII}$-Verbindung wie Cernitrat, Kaliumpermanganat oder Vanadiumpentoxid, zweckmäßigerweise in Gegenwart eines Reaktionsbeschleunigers wie einem Fe-, Cu-, Mn- oder V-Salz, z. B. in Gegenwart von Kupfersulfat, Eisen-II-chlorid, Mangan-II-acetat oder Vanadylsulfat, vorzugsweise in einem pH-Bereich von 4.0—8.5 und bei Temperaturen zwischen 0 und 100°C, vorzugsweise jedoch bei Temperaturen zwischen 20 und 35°C, durchgeführt. Hierbei kann die Aktivität der Metallionen mit einem Komplexbildner wie Äthylendiaminotetraessigsäure, Nitrilotriessigsäure oder Ascorbinsäure oder einem Kronenäther, z. B. 18-Krone-6, gesteuert werden. Besonders vorteilhaft wird die Umsetzung in Gegenwart eines Puffers wie einem Phosphatpuffer und mit einem 1,1—3,5fachen Überschuß der eingesetzten Peroxyverbindung durchgeführt.

Die Umsetzung kann auch enzymatisch durchgeführt werden, z. B. unter Verwendung von Hydroxylasen, Monoxygenasen oder Peroxidasen.

Die erfindungsgemäß erhaltenen Verbindungen der allgemeinen Formel I sowie ihre 3H-Tautomeren lassen sich in ihre physiologisch verträglichen Säureadditionssalze mit anorganischen oder organischen Säuren überführen. Als Säuren kommen beispielsweise Salzsäure, Bromwasserstoffsäure, Schwefelsäure, Milchsäure, Zitronensäure, Weinsäure, Maleinsäure oder Fumarsäure in Betracht.

Die bei dem Verfahren verwendeten Ausgangsstoffe sind literaturbekannt. Diese erhält man beispielsweise durch Umsetzung von 2,3-Diamino-pyridin mit einem entsprechenden Benzoesäurederivat unter Ringschluß und gegebenenfalls anschließender Oxidation.

Wie bereits eingangs erwähnt, weisen die neuen Verbindungen sowie ihre physiologisch verträglichen Säureadditionssalze wertvolle pharmakologische Eigenschaften auf, insbesondere bei einer geringen Wirkung auf den Blutdruck und die Herzfrequenz eine positive inotrope Wirkung.

Beispielsweise wurden die Verbindungen

A = 2-(2-Methoxy-4-methylsulfinyl-phenyl)-6-hydroxy-1H-imidazo-[4,5-b]pyridin und
B = 2-(2-Methoxy-4-methylsulfonyl-phenyl)-6-hydroxy-1H-imidazo-[4,5-b]pyridin

auf ihre biologischen Eigenschaften wie folgt untersucht:

2–3 Katzen beiderlei Geschlechts, Körpergewicht 3,2 bis 3,6 kg, wurden mit 40 mg/kg i. p. Pentobarbital-Natrium narkotisiert. Die Trachea wurde eröffnet, und die Tiere atmeten während des Versuchs spontan durch eine Trachealkanüle. Von der rechten Arteria carotis aus wurde ein Kathetertipmanometer (Millar Mikrotip P-350 A) in die linke Herzkammer eingeführt. Aus dem damit gewonnenen Ventrikeldruck wurden die Kontraktilitätsparameter $dp/dt_{max}$ und $V_{CE}$ mit Analogrechnern ermittelt. Mit Hilfe eines Statham Druckwandlers (P 23 Dc) und eines in die Bauchaorta eingeführten Katheters wurde der arterielle Blutdruck gemessen. Die Herzfrequenz wurde mit einem durch das EKG gesteuerten Tachographen ermittelt. Alle Größen wurden fortlaufend auf einem Hellige Direktschreiber aufgezeichnet.

Die zu untersuchenden Substanzen wurden 0,1%ig (Dosis: 0,1 bzw. 0,3 mg/kg i. v.) und 1%ig (Dosis: 1,0 bzw. 3,0 mg/kg i. v.) in einem Gemisch aus Polydiol 200 und 0,9%iger Kochsalzlösung gelöst. Alle Substanzen wurden kumulativ dosiert im Abstand von 10 Minuten. Die Gesamtdosen sind in der nachfolgenden Tabelle angegeben:

| Substanz | Dosis mg/kg i. v. | Änderung Blutdruck mm/Hg | Änderungen in % dp/dtmax | $VC_E$ | Herzfrequenz |
|---|---|---|---|---|---|
| A | 0,1 | 0 | + 10,8 | +12,1 | + 1,5 |
| | 0,3 | 0 | + 22,4 | +19,8 | + 1,5 |
| | 1,0 | 0 | + 32,4 | +28,9 | + 1,5 |
| | 3,0 | 0 | + 66,6 | +48,6 | + 4,7 |
| B | 0,1 | + 3/5 | + 13,9 | +13,4 | 0 |
| | 0,3 | + 8/8 | + 41,5 | +34,6 | + 5,6 |
| | 1,0 | +10/8 | + 79,8 | +60,6 | + 9,7 |
| | 3,0 | +13/5 | +116,8 | +96,1 | +10,4 |

Ergänzend sei hier noch darauf hingewiesen, daß beide Substanzen auch bei der höchsten applizierten Dosis keinerlei toxische Nebenwirkungen zeigten. Außerdem weisen die neuen Verbindungen eine wesentlich polarere Struktur als die eingesetzte Ausgangsverbindung auf. Sie werden daher leicht, ohne zusätzliche Biotransformation, eliminiert, das heißt, die Substanzbelastung des Organismus ist äußerst gering, insbesondere da durch den Hydroxysubstituenten die Epoxidbildung im Organismus verhindert wird.

Aufgrund ihrer pharmakologischen Eigenschaften eignen sich die erfindungsgemäß hergestellten Verbindungen der allgemeinen Formel I und deren 3H-Tautomere sowie deren physiologisch verträglichen Säureadditionssalze mit anorganischen oder organischen Säuren zur Behandlng von Myokardinsufficienzen und lassen sich hierzu, gegebenenfalls in Kombination mit anderen Wirksubstanzen, in die üblichen Zubereitungsformen wie Tabletten, Dragées, Kapseln, Suppositorien, Suspensionen, Tropfen oder Ampullen einarbeiten. Die Einzeldosis beträgt hierbei am Erwachsenen 35 bis 250 mg, vorzugsweise 50 bis 100 mg, 2- bis 4mal täglich.

Die nachfolgenden Beispiele sollen die Erfindung näher erläutern:

Beispiel 1

2-(2-Methoxy-4-methylsulfinyl-phenyl)-6-hydroxy-1H-imidazo-[4,5-b]pyridin und

2-(2-Methoxy-4-methylsulfonyl-phenyl)-6-hydroxy-1H-imidazo-[4,5-b]pyridin

7,2 g 2-(2-Methoxy-4-methylsulfinyl-phenyl)-1H-imidazo[4,5-b]pyridin werden in Phosphatpuffer pH 7,2 und 120 ml 3%igem Wasserstoffperoxid gelöst. Hierzu gibt man eine Lösung von 9,3 g Äthylendiaminotetraessigsäure, 4,4 g Ascorbinsäure, 3,8 g Eisen(II)sulfat in Phosphatpuffer pH 7,2. Nach 30 Minuten wird im Vakuum eingeengt, der Rückstand durch Chromatographie über eine Polystyrol-Harz-Säule (XAD-2 der Firma Röhm & Haas) mit den Elutionsmitteln Wasser und Methanol gereinigt. Nach dem Einengen des Eluats werden die beiden Verbindungen mittels Chromatographie an Kieselgel (Elutionsmittel: Chloroform/Methanol/Ammoniakwasser = 85/15/2 (v/v)) voneinander getrennt. Man erhält 2-(2-Methoxy-4-methylsulfinyl-phenyl)-6-hydroxy-1H-imidazo-[4,5-b]pyridin als gelbes Pulver vom Schmelzpunkt 220—223° C.

| | | | | |
|---|---|---|---|---|
| Ber.: | C 55,45 | H 4,29 | N 13,86 | S 10,56 |
| Gef.: | C 54,10 | H 4,49 | N 13,74 | S 11,15 |

Man erhält 2-(2-Methoxy-4-methylsulfonyl-phenyl)-6-hydroxy-1H-imidazo-[4,5-b]pyridin als gelbes Pulver vom Schmelzpunkt 250° C (Zers.).

| | | | | |
|---|---|---|---|---|
| Ber.: | C 52,66 | H 4,08 | N 13,17 | S 10,03 |
| Gef.: | C 52,15 | H 4,34 | N 13,27 | S 11,45 |

Beispiel I

Tabletten zu 100 mg 2-(2-Methoxy-4-methylsulfinyl-phenyl)-6-hydroxy-1H-imidazo-[4,5-b]pyridin

Zusammensetzung:

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

| | |
|---|---|
| Feuchtsiebung: | 1,5 mm |
| Trocknen: | Umlufttrockenschrank 50° C |
| Trockensieben: | 1 mm |

Dem Granulat die restlichen Hilfsstoffe zumischen und Endmischung zu Tabletten verpressen.

| | |
|---|---|
| Tablettengewicht: | 175 mg |
| Stempel: | 8 mm Ø |

Beispiel II

Dragées zu 50 mg 2-(2-Methoxy-4-methylsulfinyl-phenyl)-6-hydroxy-1H-imidazo-[4,5-b]pyridin

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm |
| Trocknung: | 50° C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

| | |
|---|---|
| Dragéegewicht: | 120 mg |

## Beispiel III

Suppositorien zu 75 mg 2-(2-Methoxy-4-methylsulfinyl-phenyl)-6-hydroxy-1H-imidazo-[4,5-b]pyridin

1 Zäpfchen enthält:

| | |
|---|---|
| Wirksubstanz | 75,0 mg |
| Zäpfchenmasse (z. B. Witepsol H 19) und Witepsol W 45) | 1625,0 mg |
| | 1700,0 mg |

### Herstellungsverfahren

Die Zäpfchenmasse wird geschmolzen. Bei 38° C wird die gemahlene Wirksubstanz in der Schmelze homogen dispergiert. Es wird auf 35° C abgekühlt und in vorgekühlte Suppositorienformen ausgegossen.

| | |
|---|---|
| Zäpfchengewicht: | 1,7 g |

## Beispiel IV

Ampullen zu 50 mg 2-(2-Methoxy-4-methylsulfinyl-phenyl)-6-hydroxy-1H-imidazo-[4,5-b]pyridin

1 Ampulle enthält:

| | | |
|---|---|---|
| Wirksubstanz | | 50,0 mg |
| Sorbit | | 250,0 mg |
| Dest. Wasser | ad | 5,0 ml |

### Herstellungsverfahren

Die Wirksubstanz und Sorbit werden in dest. Wasser gelöst, dann wird auf das angegebene Volumen aufgefüllt und sterilfiltriert.

| | |
|---|---|
| Abfüllung: | in Ampullen zu 5 ml |
| Sterilisation: | 20 Minuten bei 120° C |

Beispiel V

Tropfen mit 250 mg pro 5 ml 2-(2-Methoxy-4-methylsulfinyl-phenyl)-6-hydroxy-1H-imidazo-[4,5-b]pyridin

| | | |
|---|---|---|
| Wirksubstanz | | 5,0 g |
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Äthanol | | 40,0 g |
| Dest. Wasser | ad | 100,0 ml |

Herstellungsverfahren

Die Benzoesäureester werden in Äthanol gelöst und anschließend das Anisöl und das Menthol zugegeben. Dann wird die Wirksubstanz, Glycerin und Saccharin-Natrium in Wasser gelöst zugegeben. Die Lösung wird anschließend klar filtriert.

**Patentansprüche**

1. 6-Hydroxy-2-phenyl-imidazo[4,5-b]pyridine der allgemeinen Formel

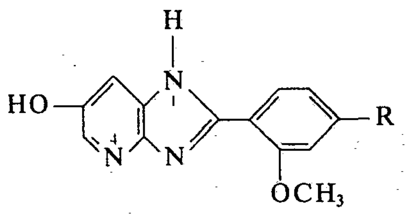

(I)

in der

R eine Methylsulfinyl- oder Methylsulfonylgruppe darstellt,

deren 3H-Tautomere und deren physiologisch verträgliche Säureadditionssalze mit anorganischen oder organischen Säuren.

2. 2-(2-Methoxy-4-methylsulfinyl-phenyl)-6-hydroxy-1H-imidazo-[4,5-b]pyridin und dessen Säureadditionssalze.

3. 2-(2-Methoxy-4-methylsulfonyl-phenyl)-6-hydroxy-1H-imidazo-[4,5-b]pyridine und dessen Säureadditionssalze.

4. Arzneimittel, enthaltend eine Verbindung gemäß den Ansprüchen 1 bis 3 neben gegebenenfalls einem oder mehreren inerten Trägerstoffen und/oder Verdünnungsmitteln.

5. Verwendung einer Verbindung gemäß den Ansprüchen 1 bis 3 zur Herstellung eines Arzneimittels mit einer cardiotonischen Wirkung auf nichtchemischem Wege.

6. Verfahren zur Herstellung von 6-Hydroxy-2-phenyl-imidazo-[4,5-b]pyridinen der allgemeinen Formel

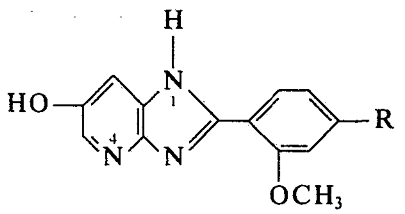

(I)

in der

R eine Methylsulfinyl- oder Methylsulfonylgruppe darstellt,

und von deren 3H Tautomeren und deren physiologisch verträglichen Säureadditionssalzen mit anorganischen und organischen Säuren, dadurch gekennzeichnet, daß eine Verbindung der allgemeinen Formel

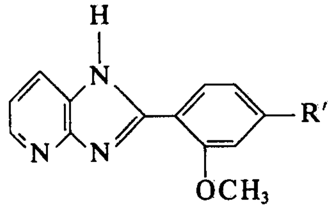

(II)

in der

R' eine Methylmercapto-, Methylsulfinyl- oder Methylsulfonylgruppe darstellt,

mit einem Oxidationsmittel oder enzymatisch kernhydroxyliert wird und gewünschtenfalls anschließend eine so erhaltene Verbindung der allgemeinen Formel I oder 3H Tautomeres in ihr physiologisch verträgliches Säureadditionssalz mit einer anorganischen oder organischen Säure übergeführt wird.

7. Verfahren gemäß Anspruch 6, dadurch gekennzeichnet, daß die Umsetzung in einem Lösungsmittel und bei Temperaturen zwischen 0 und 100° C, vorzugsweise jedoch bei Temperaturen zwischen 20 und 35° C, durchgeführt wird.

8. Verfahren gemäß den Ansprüchen 6 und 7, dadurch gekennzeichnet, daß die Umsetzung in Gegenwart eines Puffers in einem pH-Bereich von pH 4 bis 8,5, vorzugsweise jedoch von pH 7,0—7,5, durchgeführt wird.

9. Verfahren gemäß den Ansprüchen 6 bis 8, dadurch gekennzeichnet, daß die Umsetzung in Gegenwart eines Komplexbildners und/oder Reaktionsbeschleunigers durchgeführt wird.

**Claims**

1. 6-Hydroxy-2-phenyl-imidazo[4,5-b]pyridines of general formula

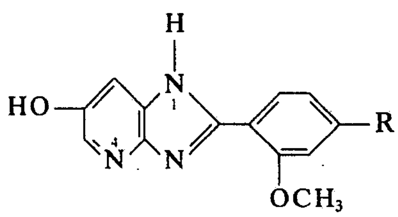

(I)

wherein

R represents a methylsulfinyl or methylsulfonyl group,

the 3H-tautomers thereof and the physiologically acceptable acid addition salts thereof with inorganic or organic acids.

2. 2-(2-Methoxy-4-methylsulfinyl-phenyl)-6-hydroxy-1H-imidazo[4,5-b]pyridine and the acid addition salts thereof.

3. 2-(2-Methoxy-4-methylsulfonyl-phenyl)-6-hydroxy-1H-imidazo[4,5-b]pyridine and the acid addition salts thereof.

4. Pharmaceutical compositions containing a compound as claimed in claims 1 to 3, optionally together with one or more inert carriers and/or diluents.

5. The use of a compound as claimed in claims 1 to 3 for the preparation of a pharmaceutical composition with a cardiotonic activity by a non-chemical method.

6 A process for the preparation of 6-hydroxy-2-phenyl-imidazo[4,5-b]pyridines of general formula

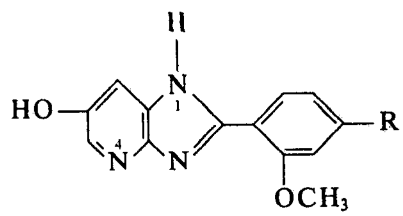

(I)

wherein

R represents a methylsulfinyl or methylsulfonyl group,

and of the 3H-tautomers thereof and of the physiologically acceptable acid addition salts thereof with inorganic or organic acids, characterised in that a compound of general formula

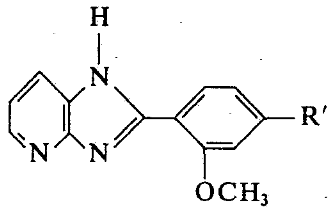

(II)

wherein

R′ represents a methylmercapto, methylsulfinyl or methylsulfonyl group,

is hydroxylated at the nucleus with an oxidizing agent or enzymatically, and, if desired, a compound of general formula I thus obtained or the 3H tautomer thereof is subsequently converted into the physiologically compatible acid addition salt thereof with an inorganic or organic acid.

7. A process as claimed in claim 6, characterised in that the reaction is carried out in a solvent and at temperatures between 0 and 100° C, but preferably at temperatures between 20 and 35° C.

8. A process as claimed in claims 6 and 7, characterised in that the reaction is carried out in the presence of a buffer in a pH range of from 4 to 8.5, but preferably from pH 7.0 to 7.5.

9. A process as claimed in claims 6 to 8, characterised in that the reaction is carried out in the presence of a complexing agent and/or a reaction accelerator.

**Revendications**

1. 6-hydroxy-2-phényl-imidazo[4,5-b]pyridines de formule générale

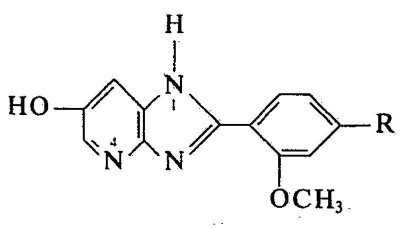

(I)

dans laquelle

R représente un groupe méthylsulfinyle ou méthylsulfonyle,

leurs tautomères 3H et leurs sels d'addition d'acides physiologiquement supportables avec des acides minéraux ou organiques.

2. La 2-(2-méthoxy-4-méthylsulfinyl-phényl)-6-hydroxy-1H-imidazo-[4,5-b]pyridine et ses sels d'addition d'acides.

3. La 2-(2-méthoxy-4-méthylsulfonyl-phényl)-6-hydroxy-1H-imidazo-[4,5-b]pyridine et ses sels d'addition d'acides.

4. Médicament contenant un composé selon les revendications 1 à 3 de façon éventuelle conjointement à un ou plusieurs excipients et/ou diluants inertes.

5. Utilisation d'un composé selon les revendications 1 à 3 pour la préparation d'un médicament avec une activité cardiotonique par voie non chimique.

6. Procédé pour la préparation de 6-hydroxy-2-phénylimidazo[4,5-b]pyridines de formule générale

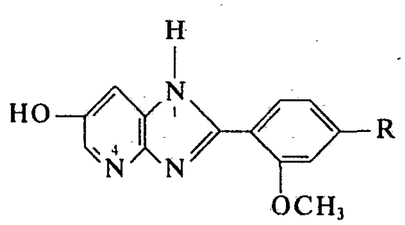

(I)

dans laquelle

R représente un groupe méthylsulfinyle ou méthylsulfonyle,

et de leurs tautomères 3H et de leurs sels d'addition d'acides physiologiquement supportables avec des acides minéraux et organiques, caractérisé en ce qu'on hydroxyle dans le noyau, au moyen d'un agent d'oxydation ou enzymatiquement, un composé de formule générale

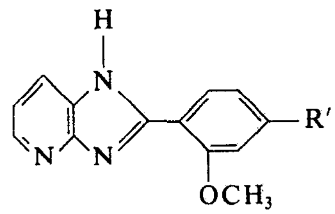

(II)

dans laquelle

R' représente un groupe méthylmercapto, méthylsulfinyle ou méthylsulfonyle

et en ce qu'éventuellement on transforme ensuite un composé ainsi obtenu de formule générale I ou son tautomère 3H en son sel d'addition physiologiquement supportable avec un acide minéral ou organique.

7. Procédé selon la revendication 6, caractérisé en ce que la réaction est effectuée dans un solvant et à des températures entre 0 et 100° C, de préférence cependant à des températures entre 20 et 35° C.

8. Procédé selon les revendications 6 et 7, caractérisé en ce que la réaction est effectuée en présence d'un tampon dans un domaine de pH de 4 à 8,5, de préférence cependant de pH 7,0—7,5.

9. Procédé selon les revendications 6 à 8, caractérisé en ce que la réaction est effectuée en présence d'un agent formant des complexes et/ou d'un accélérateur de réaction.